# EUROPEAN PATENT APPLICATION

(11) **EP 3 249 362 A1**
(43) Date of publication of application: **29.11.2017**
(21) Application number: 15878639.2
(22) Date of filing: 21.01.2015
(51) Int. Cl.: G01C 22/00

(54) **INSOLE WITH INTEGRATED NANO-PEDOMETER, STEP DETECTION AND COUNTING METHOD USING SAID INSOLE, AND SHOE EQUIPPED WITH THE FIXED OR REMOVABLE INSOLE**

(71) Applicant: Multiservicios Profesionales De Esparza, S.A., Esparza, Puntarenas (CR)
(72) Inventor: CAMPOS GALLO, Allan, Pavas San José (CR); CAMACHO ELIZONDO, Karen Melissa, Pavas San José (CR); VEGA BAUDRIT, José, Pavas San José (CR); NUÑEZ AGUILAR, David, Pavas San José (CR)
(74) Representative: Carvajal y Urquijo, Isabel
(86) International application number: PCT/CR2015/000001
(87) International publication number: WO 2016/116071

(57) **Abstract**

An insole with a nanopedometer for step counting and the measurement of information parameters obtained by the same device, as well as a shoe or sneaker with said insole.

## Description

### TECHNOLOGICAL AREA

This invention is related to the health area and to the biomechanical engineering industry and it specifically refers to an insole with a nanopedometer for step counting and the measurement of information parameters obtained by the same device, as well as a shoe or sneaker with said insole.

### INVENTION BACKGROUND

According to the World Health Organization (WHO) document "Global Recommendations on Physical Activity for Health" for both 2005 and 2007 unhealthy eating and lack of physical activity stand out among the leading causes of the most important non-communicable diseases and affects a significant percentage of mankind today. WHO estimates that two million people die due to lack of physical activity.

Nationally there has been a serious rise in both childhood and adult obesity. Indeed it is projected that if the current pace is maintained, in a few decades, Costa Rica will be one of the countries with the largest obesity problems in America.

According to the California Board of Pediatric Medicine, one of the most popular ways of exercising is walking, because it is easy as it is safe and low cost. This improves heart health as well as muscular and psychological health. However, it is not only to walk but to do it in a controlled manner, wear proper clothing and following some follow up and progressive improvement system.

According to the latest studies, the gradual increase of the daily walk has a direct result in an 8% decrease of heart risk, and multiple improvements on the health of people with diabetes, estimating that said improvement could reach up to a 50% in the decrease of cardiac risk and lesions, if this routine is kept 3 to 5 times a week.

The ideal goal recommended by the American Heart Association and the British Heart Foundation is of 10,000 daily steps (equivalent to 8 kilometers). However, several specialists indicate that walking is not the only possibility to carry out physical activities and point out that, as an example, a walk at the mall or at other places are an equivalent. As a matter of fact, a stroll along the street is an estimated 500 steps, going to the supermarket 1550 steps and walking the dog 4200 steps. The most important thing is to keep an accurate track of the steps given.

An exponentially accelerated growth has been generated in the scientific analysis of human movement and biomechanics. The term kinesiology (science of movement) was used to describe the body of knowledge related to the structure and function of the muscle-skeletal system of the human body. Further on the study of the mechanical principles applied to the human movement came to be widely accepted as an integral part of kinesiology. Later on the term was used in a much more literal way to highlight aspects of all the sciences that somehow have something to do with human movement, surpassing the term kinesiology itself. Many new terms were suggested for its substitution: anthropo-mechanics, anthropo- kinetic, biodynamic, and bio kinetic or kinetic anthropology. They were all gathered by biomechanics which gained a wide acceptance.

Biomechanics has been defined in many ways. Among them are: - The mechanical basis of biology, muscle activity, the study of the principles and relations implicated; - The application of mechanical laws to live structures, especially the human body locomotor system;- It is the science that examines the inner and outer strengths that act on the human body and the effect that they produce.

The first forefathers of biomechanics were: Aristotle, Archimedes and Galen, who contributed mathematical, mechanical and anatomical paradigms that instrumental in the development of biomechanics.

During the Italian Renaissance, the Scientific Revolution and the Enlightenment, scientists such as: Leonardo Da Vinci, Vesalius, Galileo Galilei, Giovanni Alfonso Borelli, Sir Isaac Newton, Leonard Euler or Joseph-Louis Lagrange provided the foundations of modern biomechanics, through the study of the concept of strength, muscle action and the law of mechanical motion.

In the 19^{th} century several different instruments to study movement were developed, such as the photographic camera (first technological device used in the study of human movement) and devices like instrumented soles to assess foot pressure during gait o while running and the dynamometer platform, considered the first force platform.

The implementation of biomechanics to sports had a more recent origin (at the end of the 19^{th} 10 century) when the first 3D human movement analysis were done, calculating speeds and acceleration of different body segments.
On the 20^{th} century the body mechanics was studied, oriented to the world of work, using dynamometer tracks that would register force in four directions. Additionally, deep studies of muscle physiology were done. Starting in the 1960's computers made the data analysis, of data registered by the cameras and force platforms, easier in the biomechanical studies.

As of the 1970's the research of sports biomechanics have been based on various technics such as cinematography, electromyography, goniometry, force platforms and computer simulation and more recently, data collection from devices worn by athletes such as pedometers.

### Human movement classification:

Human movement can be classified according to the following scheme: Medical Biomechanics which evaluates the pathologies that afflict man in order to generate solutions capable of evaluating, repairing or alleviating them. The Physiotherapeutic Biomechanics evaluates the musculoskeletal systems dysfunction in the human being, so it can observe, evaluate, treat or diminish said dysfunctions. The physiotherapeutic biomechanics addresses anatomy from a functional point of view, following international anatomic terms in time function. The sports Biomechanics analyzes sport practice in order to improve its performance, develop training technics and design complements, materials and high performance equipment. It focuses on the research of sport specific technics, design the best sport equipment, clothing and identifies the practices that are prone to lesions. Occupational Biomechanics studies the interaction of the human body with the elements it relates to in various areas, in order to adapt them to its needs and capabilities.

Much of the knowledge generated by biomechanics are based on biomechanical models which allow to forecast behavior, resistance, fatigue and other aspects of different parts of the body when it is subject to certain conditions. Among the most currently used technics we can mention the photogrammetry analysis which consists of 3D-movement based on digital video. The application provides information regarding three-dimensional movement of people and objects in space which images have been captured and processed. In the same way it is possible to apply the tension-deformity behavior analysis directly, which determines the resistance of a biological material when force is applied onto it. It can be a compressive or tensile force and it generates fundamental changes in the structure. Lastly, it is possible to apply computational biomechanics which refers to computer simulations of biomechanical systems to test and refine theoretical models as well as for technical applications.
In the state of the art, the combination of all of these elements had not been applied, which precisely are the ones that lead to this invention and thanks to which an important inventive leap takes place in regards to previous art in order to design the nanopedometer.

Important precedents exits, worthy of being pointed out as it describes previous technologies that can be taken as prior to the current invention and on which an important technological leap was achieved, transforming the technology in order to obtain an invention that is able to comprehensively combine the interpretations of the elements derived from biomechanics.

Invention US6807869 B2 displays a sensor that detects the presence of a force exercised by the foot of a person, located between the foot and the ground, in the region of the shoe sole. The sensor includes an elastic comprehensible intermediate elastomeric mat of material placed in between a lower conductive layer and a multiplicity of upper portions of the layer of the **30** insulating conductive cloth

The layer of elastomeric elastic includes a multiplicity of electrically conductive metal strands that extend from a first surface to a second surface of the elastomer layer.

Although true, this precedent incorporates advanced technology, it falls short regarding the pedometer described in this body, since the piezoelectric systematic sensory distribution is much more effective when collecting thrust. Additionally the piezoelectric and piezoceramic technology included in this pedometer, provides a more precise signal detection and transmission to the processing unit.

In the application CN 203059714 U, the invention incorporates a pressure collection module strategically positioned and used for foot pressure control, a wireless communication module used to transmit data processed by the real time data processing module and in a wireless fashion.

The pressure collection module contains a multiplicity of pressure micro sensors that are located at the first metatarsal head, at the head of the fifth metatarsal and in an area of the anatomy of the heel.
The preceding paragraph identifies one of the aspects that differentiates this pedometer from its predecessor, as the location of the sensor is one of the other comparative advantages that it has over others. A thorough review of previous studies managed to identify the exact locations that allow the collection without omissions nor distortions, therefore the sensor location as well as the processing unit, the interconnection base and microcontroller accurately guarantee the information transduction.

Additionally, the internal analysis within the processing unit, of the motion patterns in order to identify whether or not there are steps, which is another point that stands out from the rest of the inventions that were taken as precedents in order to make the previous review.

### DETAILED DESCRIPTION OF THE INVENTION

### 1. Research background

This invention consists of an insole with a nanopedometer used to count steps and measure the information parameters obtained via the same device, as well as a shoe or sneaker with said insole. Developed after intense research that determined the specific factors that the device should comply with for its optimum operation.

The pedobarographs (based on a technic used for the first time by Chodera in 1957) are optical systems that measure the foot pressure. They need to process images of the sole of the foot in order to obtain results, which requires high computer power in order to obtain short term results.

This has been favored by the increase of computer capacity, with an affordable cost for the majority of medical and educational centers, and there are many applications that allow real time high processing of high resolution images and video.

Different methods are used for the study of the foot, from the morphological and biomechanical point of view, including notable ones such as the kinetic, radiological, photographic, hypodynamic, Shulzhenko methodology, tens metric, Strain amplifier, computerized charge oscillography and the pedography. These methods have allowed us to study the foot in order to classify and determine the modifications that occur in it.

The trajectory of the application point, resulting from body mass, starts at the moment of impact in the central zone of the heel. Afterwards it quickly moves in a straight line to the first and second metatarsal heads, turns inwards and slowly moves to the base of the first toe. It ends at the tip of the first or second toe, or in between them.

When walking, at the beginning (contact phase) a maximum vertical force is observed (Fz) The heel is in contact with the ground, which reduces the forward move of the foot. This implies a backwards reaction. Given the starting position of the foot in supination, the anterior-posterior forces (Fx) are negative and as the pronation movement starts the Fx forces get to zero and then turn positive up until the contact of the foot ends.

Hereafter (support phase) a drop of the vertical force occurs. This happens when the foot is flat and it corresponds to the change of the braking period of the previous thrust. During this period the foot does not develop transversal forces (Fy) but it must insure transversal stability. Finally, during the thrust phase, the forces have an upward and forward orientation.

The ground reaction forces are frequently expressed as normalized to the body weight (BW). The forces observed when running are superior to the ones showed when walking. Normally the maximum force peak Fz in the heel of the foot when walking, varies between 2.5 and 4 BW, although in some instances it could be higher: for example while playing basketball, when 10 jumping the impact vertical force can reach from 7 to 9 BW. When walking, Fz varies from 1 to 1.5 BW.

There are many factors that affect the maximum force, such as locomotion, speed, the ground, as well as parameters relating to the subject such as: body weight, age, pathologies... The study of these forces makes it possible to locate zones (including the application point of the forces) that show higher pressure values and also quantify these values.

Two types of mechanical effort can be singled out: a) *force applied* in a vertical way over the plantar surface, and b) *force parallel* or shearing force to the plantar surface.

It was determined in the research that a difference exists between measured and real pressure distribution. The real pressure is the local force under the foot or total force of the foot structure through the skin and soft tissues over the foot sole area. Measured pressure is the local force over the measurement area of the sensor or sensors.

The differences between the *real and measured* pressures are caused by: differences between the real sole area and the area covered by sensors, accuracy of the measuring sensor, dimensions of the measuring sensors, number of sensors, sensor distribution in the measuring zone, measurement frequency (number of values per second), properties of the measuring zone (Rigid or flexible, thick or thin).

The distribution of pressures in a person when walking depends on the way the foot is flexed and lifted from the surface, the way the person walks (repeatedly, steady, insecure, forced, quick, slow, straight, curvy) the anatomy and psychology of the person and diseases and disorders.

Various sensors have been developed to measure the maximum pressure over the contact surface. The most utilized ones are the electric pressure sensors and the pneumatic sensors.

The electric pressure sensors consist in various electrical measuring cells that work depending on resistance variation and the capacity or induction of the sensor caused by pressure changes. Among them are the resistive, the piezo resistive, the capacitive, the piezoelectric and the force sensor.

The resistive and the piezo resistive have an inconvenient, since the material must be very soft in order to insure the pressure sensitivity. However the behavior of soft materials often is time- dependent (viscoelasticity).
On the other hand, pneumatic sensors start registering pressure as soon as the internal and external pressure of the inflatable cells has reached a balance. They are insensible to shearing forces and temperature, they are relatively cheap, can be made into various sizes without manipulating its thickness, they're thin, flexible and easy to use. The advantage of the flexibility: diminishes due to a change in the sensors response if located on a surface with a conspicuous curve. The inconvenient is that measurement frequency is very low, around 5 Hz max.

Different measuring systems were analyzed to assess the pressure/force distribution. Determined that the rigid system with force platforms allows to measure the normal and shearing forces to the applied load. It is used, among other uses, to locate the projection of the body's center of gravity. On the other hand the flexible system, like the pneumatic mats, serves to measure the same parameters, but show as an inconvenient that they distribute the pressure to be monitored, therefore interfering with the real pressure values, they do no measure the shearing force and it is an static measure (the immediate pressure is registered instead of the time-related pressure changes).

The insoles are used in applied and clinical research. Usually the measurement is done during a very limited period of time, generating a large amount of data in a short period of time. Some systems use cables which greatly limits the utilization area.

An important aspect to be considered is that the pressure forces are measured in the interface between the skin and the surface and not on the tissue itself.

The distribution of pressure in the different areas depends greatly on the measuring system used. Discussion is still going about which of the systems is closer to the real distribution of pressure. They all seem to provide valid results in a qualitative manner. The resistant sensors tend to show relatively high sensibility changes and movements.

Within the research, a study regarding plantar pressures was also done. Plantar pressures in a static condition, were shown in our invention (bipedal support) because of the body weight that is transmitted throughout both of the lower extremities reaching each with a 50% of its total value. The first bone of the foot, (astragalus), distributes this force to its points of support. The static foot presents two triangles, a posterior or support one which goes from the calcaneus to the head of the metatarsals and another one, anterior or drive which is made from the metatarsals and the toes.

Many authors have determined that, in static, there is a major load on the heel. However, the weight distribution is hard to calculate due to the fact that in one person it significantly varies due to the normal balance of the body ("dynamic standing"). The balance makes that the weight that different zones support will vary constantly and this distribution also depends on the position of the foot. Additionally, it is important to note that the methodology used in the different studies that have taken place to register plantar pressures also influence also in that they can be very varied.

Almost all studies coincide that the pressure on the heel is greater than on the rest of the foot and that the external band of the middle of the foot the pressure values are very low. In the forefoot, when loading, a descent of the transverse arch takes place, with the support of the heads of the five metatarsals.

Toes offer a relatively weak support, but on the big toe greater pressure values are observed. During static support, the first toe supports a 97% of the study subjects, taken the largest pressure from them all, although certain researchers make no difference between the big toe and the rest of the toes, having found a similar value.

If it is a uniped support, all of the points of the foot will be subject to a greater pressure since load is not distributed. The posterior heel is overloaded and the pressure is greater than double of its value in bipedal support and the external band on the midfoot increases its width and its pressure. On the forefoot the pressures will also elevate in relation with the bipedal support but in an even way between all metatarsals, although a recent study pointed out that, in a monopod position, the load is spread between the first and forth. The toes show a higher pressure in a monopod support due to, besides the load increase, the work of the flexor muscles, required by the swaying of the foot that carry out the duty of ground gripping and stabilization.

In regards to plantar pressure in gait, many studies exist regarding pressure distribution in gait, which present many different values. This happens due to the different methods and technics used in each one of them. Generally, the weight that reaches the ground while in gait is not even but it varies in waves, with two pressure peaks one of them coincides with heel contact and the other one with the start.

The authors divide the sole of the foot considering the areas of greater biomechanical interest. Generally, it is considered that the maximum local pressure of the entire foot is registered on the heel and this takes places with its initial contact to the ground. Certain authors contradict this by indicating that the main support zone is the forefoot. Additionally, some literature was found that describes that the maximum pressure occurs at the time that it also contacts the lateral edge of the metatarsal heads. Everyone agrees that as the body weight moves to the central zone of the heel and the support surface increases, pressure starts decreasing.

The pressure values in different studies are very variable, although it does indicate that the pressure zones are very similar to the ones found in static. The differences are the new areas that have appeared and the increase of the surface of the central zone of the posterior heel.

It has been determined that in gait, after the initial support of the lateral metatarsal heads, a progressive liberation of these takes places so that the medial reach the ground in which way so that the phase in which the toes start to get off the ground can start. The majority of the researchers describes a higher pressure on the heads of the second and third metatarsal, at this point reaching between 60% and 100% of the value, on the impact in the heel. The terminal **10** support on the first metatarsal head and the big toe is essential for gait, since it constitutes the support point so that the flexor muscles can apply their driving force.

Even if it is clear that no unique plantar pressure distribution pattern exists, four normal patterns are proposed, that vary according to individual differences: The first one is a *Medial Pattern* which gives major support on the third, followed closely by the first and second metatarsal. The second one is the *Central-Medial Pattern* which gives major support at the second and third metatarsal, followed by the first one. The third one is the *Central Pattern* which gives major support at the second and third metatarsal, followed by the fourth one. Finally, the fourth one is a *central-lateral Pattern* which gives major support to the third, fourth and fifth metatarsal.

The midfoot has no role to play in weight transfer from the heel to the forefoot. However, investigation have determined that this pressure represents a 10% of the heels maximum contact.

If you would want to determine by means of an analysis of the footprint, it is not the same to do it with a static analysis than with a dynamic one, since in gait the assessment of any pathology is not very representative. The footprint is not consistent with the dynamic behavior of the foot, which could show non discernable pathologies in a static position.

Among the factors that have an effect over the distribution of plantar pressure, the weight, age, gender, speed and gait cadence stand out. An important connection exists between the weight and the plantar pressures. In overweight people, barefoot or shod, an increment on the lateral zone of the foot can be seen, especially during the mid-phase of its contact, with fewer peaks on the head of the first metatarsal. Recently, the existence of a correlation between the weight and the pressure on the head of the fourth metatarsal has been found. Heavy subjects tend to use less the medial zone of the forefoot.

The other factor is age. Precisely, gait in children has very special characteristics that evolve with the maturing of the nervous system and that stabilizes until it reaches the characteristics of adult gait. This happens, according to the majority of the authors, around seven years of age. The plantar pressures on a child are lower than on an adult, due to its lower weight and proportionally a larger pressure over the head of the first metatarsal exists, due to the knee valgus which is frequently present which leads to a greater pronation of the foot.

In the elderly (as of the age of 60-70 years old) there are variations in the gait indicators, independent from variations due to possible pathologies. It is considered a cautious gait. The foot is more horizontal in the heel contact due to a lower range of movement in the joints of the lower extremities, which conditions a diminution of the vertical reaction force and of the pressure peaks during support.

In regards to gender, many times it is not the gender what determines the distribution of the plantar pressures, but the use of different footwear and the anthropometric characteristics, such as a lower weight in women. Very similar distribution patterns are described for boys and girls. Usually the joint mobility of women is greater and this can influence that men, with greater rigidity and lower pronation on the support phase, tend to localize the greater pressure over the lateral zone of the forefoot and the toes.

Speed and cadence in gait are linked one another, since at a lower speed with the same step length, higher the per minute steps will be, in other words, cadence will be higher. It has been showed that plantar pressures are directly proportional of these factors. When speed increases, and therefore the cadence, pressure increases linearly on the heel, in the medial zone of the forefoot and on the first four toes, while there are no differences nor is pressure reduced on the lateral zone of the forefoot nor the fifth toe.

In footrace, a higher speed increases the total of the forces two to three times and therefore the pressures are also higher. Generally, when running, the highest pressure does not correspond to the heel, as it does in gait, but to the forefoot and within it is higher on the head of the second metatarsal, followed by the first and third ones.

When a study of the plantar pressures in gait takes place, it is considered that there is no need to control the speed of the person that is being evaluated but instead it is better to let them walk at their comfortable speed so it does not alter its gait pattern nor lead to a greater energy consumption.

### 2. Description of the invention

The current invention was determined after multiple investigations that the insole itself will work as support means of the following elements: 1. Piezoelectric sensors: they provide an electrical impulse depending of the pressure applied to them. 2. GPS: System through which the foot also provides information regarding location and distance travelled. 3. Power source: Small battery that will provide the necessary power for the microcontroller, GPS and transmitter and 4. Processing unit.

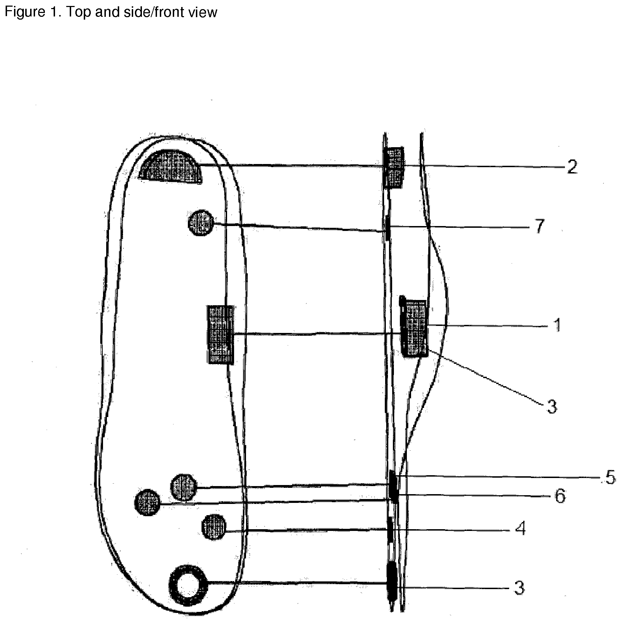

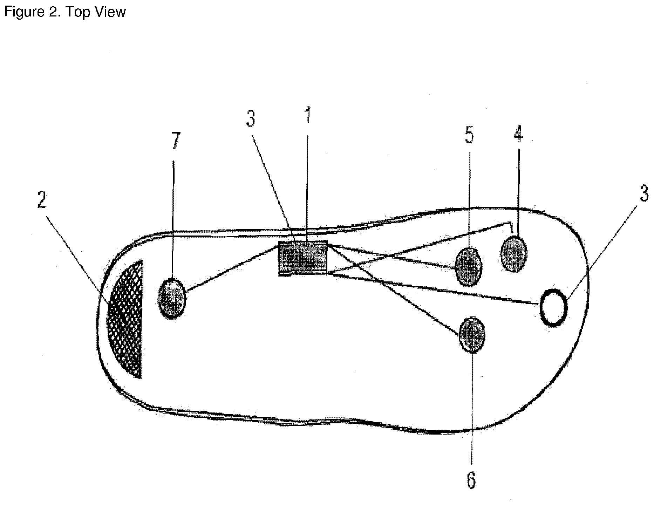

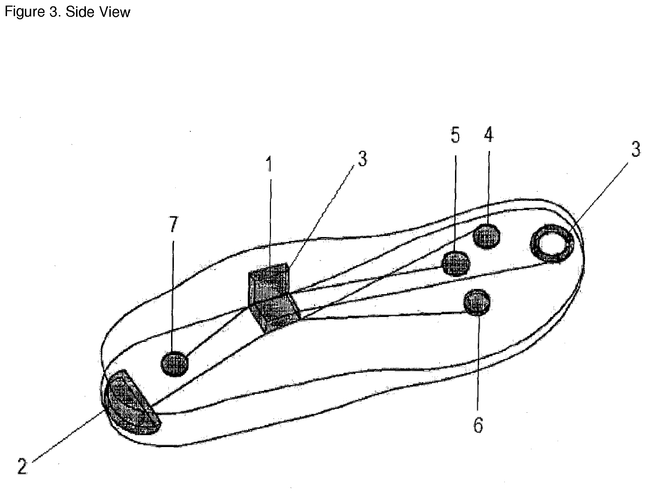

In figures 1, 2 and 3 the design of the nanopedometer insole can be seen where the figures correspond to the following component:
(1) The processing unit where the amplifier, a transmitter, a microcontroller, a power source and interconnection base are located.
(2) A power supply unit.
(3) The location device, which could also incorporate a second device.
(4) Medial Pattern piezoelectric sensor.
(5) Central-medial Pattern piezoelectric sensor.
(6) Central Pattern piezoelectric sensor.
(7) Central-lateral Pattern piezoelectric sensor.

The processing unit is made up by: 1 an amplifier: Amplifies the signal in a way that it can be interpreted correctly. 2. A transmitter: Conveys the information to an external computer/receiver via Bluetooth/Wi-Fi. 3. A microcontroller: A small computer element with programmable digital and analog inputs and outputs. 5. An electronic interconnection base: way of interconnecting all the other elements (sensors, GPS) to the power source, microcontroller and transmitter.

The piezoelectric sensors that the invention requires, are used so they can produce an impulse with sufficient breadth so it can be measured and differentiated from a non-walking activity and that can easily be amplified and converted to CD. They must be flexible, low cost, small and thin so that comfort is not compromised. Additionally they must be reliable, durable and bear small temperature and humidity changes with small electronic circuits.

It was determined that these characteristics were met by the 40 MHZ 2.0X1 piezoelectric crystals and the SMD 5031 - 32.00 MHZ pad piezoelectric crystals. Additionally instead of using said crystals, the following ceramics, or any similar material, always operating under the same principles, could be used: Standard piezoceramic disc, Lead specified with a diameter 3.00 mm and width 0.300 mm or a piezoceramic plate.

Lastly, it could be constituted by thin piezoelectric films, as long as the properties of said films are previously validated with the indicated parameters.

In order to increase precision, the use of miniature GPS is proposed. It is widely used in drones, due to its high precision and speed, aside from the fact of its miniature form. For example the Neo 7p has DGPS, that is, differential correction can be applied to the data. The differential correction must be done by a separate software, it can be personalized and in order to do it in real time a radio connection with reference antenna is needed. The EI EVA 7M from uBlox does not do differential correction but it is extremely miniaturized, measuring 7mmx7mmx1.1mm and it complies with all the functional requirements of the project.

The insole can operate with energizer lithium batteries 611322 which provide 3.00 V per unit and has an 016 x 1.4mm dimension, or also it can use a removable rechargeable battery, with the purpose that the user has two batteries that can be exchanged so that they can be recharged.

The processing unit has all the elements that allow to transduce the signal coming from the sensors and interpret it as steps. This unit is encapsulated and it is constituted by an amplifier and a microprocessor.

The load amplifier for the interface with the microcontroller allows that each transducer to have its own load amplifier. This load will be *Q* = *d₃₃oA* = *d₃₃F* where Q is load, d33 is the proper piezoelectric coefficient, A is the sensor area and F is the resultant force applied. The normal force experienced while walking is in the 200-400kPa parameter. (In certain regions a drop of 30-50 kPa is expected due to the sole of the foot effect).

The load amplifier requires an operational amplifier with a high input resistance and parasitic low-current. The layout of the load amplifying circuit is fundamental. The structure of the operational amplifier must be properly grounded and the inputs must be monitored and connected to the same ground casing. The operational amplifiers with the JFET input stage transistors are the most appropriate ones.

Regarding the transmitter, the information produced can be sent to remote stations in real time. The receiving stations could be a watch or an application (software) installed on a cell phone and/or computer or any other smart device.

In this regard, it has been decided that said communication system be based on Bluetooth technology, although it can also be via antenna and Wi-Fi. The most common and recommended option is the RDA5991 chip, which integrates the possibility of WLAN, Bluetooth and FM integration. Its dimensions (4X4X0.93 mm) make it optimal for this application, although other similar devices can be used (FUJITSU MBH7BTZ19 and BlueRadios BR-C46AR).

The Microcontroller is the signal acquisition and sampling system which must have three blocks: The initialization one: to detect the coupled systems. The measuring and sampling one: to acquire and filter the signal, and the one that save the signal to a file and transmit it to an external application or device.

The process must measure an estimative frequency since the signal is not completely periodic. Additionally the process must use the least possible amount of power the characteristics of this subsystem are that it is of low consumption, miniature size, intelligence and programming environment.

The digital processing of the signal considers four tasks: first, the A/D conversion of the analog signal, second, the calculation of the walking frequency, third, data transmission and fourth the simple user interface.

Setup takes place and the Arduino USB - LilyPad technology (or similar), over the **15** microcontroller configuration ATmega32U4 (or similar), building the printed circuit on flexible sheeting, which allows a reduction of dimensions and cost.

The processing sequence will be the following one: 1. the microcontroller receives the impulses of each piezoelectric sensor in a specific analog input. Each analog input is exclusive for a sensor. 2. Each impulse received must be filtered by intensity in order to isolate real impulses from noise. 3. Each impulse is characterized by a digital variable which is stored at the beginning of a queue along with an impulse time stamp and the identification of the sensor that originated it. 4. Stored impulses are analyzed to verify if they respond to one of the patterns recognized as steps, patterns correspond to a series of impulses, distanced by random time intervals. 5. If, among the stored impulses, a step is recognized the indicated coordinate is captured by the GPS connected to the microcontroller. 6. Afterwards, a data package with the GPS coordinate is formed, this and the time stamp is sent via the communication components of the device to a client system. 7. Once a step information is sent, all involved signals related to that step are eliminated from the impulse queue and all the previous signals.

The solution used is the impression of the circuit on flexible sheeting, which insures a personalized distribution, a minimum thickness and the possibility of having it in the insole.

### In regards to the location of the sensors

Since to date the information, provided by the company PLANTIMEC S.A. is not available, it has been arranged that the location, based on several studies, which coincide on the main regions (heel, first and second metatarsal).

Almost all the studies coincide that the pressure on the heel is greater than the one on the rest of **10** the foot and that the pressure values on the external band of the midfoot are very low. On the forefoot, when loading, a descent of the transverse arch takes place, with support from the heads of the five metatarsals.

The toes give a relatively weak support, but on the big toe, higher pressure values are observed. During static support the first toe supports, in the vast majority of subjects of different studies, receiving the highest pressure from them all.

If the support is uniped, all the points on the foot will be subject to a higher pressure since the load is not spread out. The posterior heel is overloaded and the pressure is higher than twice of its value on uniped support and the external band on the midfoot increases its width and pressure.

On the forefoot the pressures also rises in relation to the bipedal support but in an even way between all metatarsals, although a recent study showed that in a monopod position, the load is spread out between the first and fourth. The toes shoes a higher pressure on monopod support, additional to the increment of the load, the work of the flexor muscles, required by the swaying of the foot, that carry out the task of ground gripping and stabilization.

In regards to plantar pressure in gait, many studies exist regarding pressure distribution in gait, which present many different values. This happens due to the different methods and technics used in each one of them. Generally, the weight that reaches the ground while in gait is not even but it varies in waves, with two pressure peaks one of them coincides with heel contact and the other one with the start. The midfoot does not have a role to play in the weight transference from the heel to the forefoot. However, certain researchers say that the pressure in this area represents a 10% of the one from the maximum contact of the heel.

In conclusion, the invention is an insole with an integrated nano pedometer, to detect and count steps, that consists of a processing unit with an amplifier, a transmitter, a microcontroller and an interconnection base. Additionally it has a power supply unit, at least one location device and at least four piezoelectric sensors located in defined areas, for the best pressure detection.

The processing unit allows to transduce the signal coming from the sensors and interpret it as steps, based on the following components: A load amplifier for the interface with the microcontroller, a transmitter that communicates the data to an external fixed or mobile device via Bluetooth/Wi-Fi: a microcontroller characterized for it being a small computer element with programmable digital and analog inputs and outputs, and an electronic interconnection base to interconnect the power supply unit, the location device and the piezoelectric sensors to the power source, microcontroller and transmitter.

The load amplifier requires an operational amplifier with a high input resistance and parasitic low-current. The layout of the load amplifying circuit is fundamental. The structure of the operational amplifier must be properly grounded and the inputs must be monitored and connected to the same ground casing. The operational amplifiers with the JFET input stage transistors are the most appropriate ones.

Additionally, the processing unit works with a power source that consists of at least two lithium button cells arranged in a series manner up until it complies with the voltage requirement, which provide 3.00 V per unit and dimensions of 016 x 1.4mm or smaller, or a removable rechargeable battery can be used, which works as a backup device.

The power supply unit is located on the heel of the insole and it has a semicircular shape with a maximum height of 11.94 mm and a width of up to 36.66 mm.

The location device could be in the form of GPS, with a width of 7mm and a height of 1mm and that allows to get information regarding the location of the person and the distance travelled.

Concerning to the piezoelectric sensors, there made of crystal, piezoceramic disc, standard piezoceramic disc or thin piezoelectric sheets and provide an electrical impulse according to the pressure applied on them. They're circular with a diameter lower than 10.34 mm and a height or thickness lower than 1.00 mm and of a flexible material resistant to high temperatures and humidity.

The sensors could be placed according to the following distribution of the insole:
*Medial pattern,* located to give greater support to the third, closely followed by the first and second metatarsal.

*Central-medial pattern,* located to give greater support to the second and third metatarsal, followed by the first one.
*Central pattern,* located to give great support to the second and third metatarsal, followed by the fourth one.

*Central-lateral pattern,* located to give greater support to the third, fourth and fifth metatarsal.

The processing sequence occurs when the microcontroller receives the impulses of each piezoelectric sensor in a specified analog input. Each input is exclusive for a sensor. Each impulse received must be filtered by intensity in order to isolate real impulses from noise. Each impulse is characterized by a digital variable which is stored at the beginning of a queue along with an impulse time stamp and the identification of the sensor that originated it.

Stored impulses are analyzed to verify if they respond to one of the patterns recognized as steps, patterns correspond to a series of impulses, distanced by random time intervals. If, among the stored impulses, a step is recognized the indicated coordinate is captured by the GPS connected to the microcontroller

Afterwards, a data package with the GPS coordinate is formed, this and the time stamp is sent via the communication components of the device to a client system.

Once a step information is sent, all involved signals related to that step are eliminated from the impulse queue and all the previous signals.

The insole can be used in any type of shoe or sneaker in a permanent or removable manner.

## Claims

1. An insole with an integrated nanopedometer to detect and count steps that consists of:
A processing unit (1) an amplifier, a transmitter, a microcontroller and an interconnection base.
A power supply unit (2),
At least one location device (3),
At least four piezoelectric sensors (4, 5, 6, 7).

2. An insole claim 1, where the processing unit allows to transduce the signal coming from the sensors and interpret it as steps, based on the following components:
A load amplifier for the interface with the microcontroller.
A transmitter that communicates the data to an external fixed or mobile device via Bluetooth/Wi-Fi.
A microcontroller characterized for it being a small computer element with programmable digital and analog inputs and outputs.
An electronic interconnection base to interconnect the power supply unit (1), the location device (3) and the piezoelectric sensors (4, 5, 6, 7) to the power source, microcontroller and transmitter.
The load amplifier requires an operational amplifier with a high input resistance and parasitic low-current. The layout of the load amplifying circuit is fundamental. The structure of the operational amplifier must be properly grounded and the inputs must be monitored and connected to the same ground casing. The operational amplifiers with the JFET input stage transistors are the most appropriate ones.

3. An insole claim 2, where the processing unit works with a power source consisting of:
At least two lithium button cells arranged in a series manner up until it complies with the voltage requirement, which provide 3.00 V per unit and dimensions of 016 x 1.4mm or smaller, or a removable rechargeable battery can be used, which works as a backup device.

4. An insole claim 1, where the power supply unit (2) is located on the heel of the insole and it has a semicircular shape with a maximum height of 11.94 mm and a width of up to 36.66 mm.

5. An insole claim 1, where the location device (3) could be in the form of GPS, with a width of 7mm and a height of 1 mm and that allows to get information regarding the location of the person and the distance travelled.

6. An insole claim 1, where the piezoelectric sensors, there made of crystal, piezoceramic disc, standard piezoceramic disc or thin piezoelectric sheets, that are **characterized by**:
Provide an electrical impulse according to the pressure applied on them,
They're circular with a diameter lower than 10.34 mm and a height or thickness lower than 1.00 mm.
Made of a flexible material resistant to high temperatures and humidity.

7. An insole claim 1, where the sensors could be placed according to the following distribution of the insole:
*Medial pattern,* located to give greater support to the third, closely followed by the first and second metatarsal.
*Central-medial pattern,* located to give greater support to the second and third metatarsal, followed by the first one.
*Central pattern,* located to give great support to the second and third metatarsal, followed by the fourth one.
*Central-lateral pattern,* located to give greater support to the third, fourth and fifth metatarsal.

8. A method to detect and count steps by means of an insole with a, integrated nano pedometer where the information processing sequence consists of:
The microcontroller receives the impulses of each piezoelectric sensor in a specified analog input. Each input is exclusive for a sensor.
Each impulse received must be filtered by intensity in order to isolate real impulses from noise.
Each impulse is **characterized by** a digital variable which is stored at the beginning of a queue along with an impulse time stamp and the identification of the sensor that originated it.
Stored impulses are analyzed to verify if they respond to one of the patterns recognized as steps, patterns correspond to a series of impulses, distanced by random time intervals.
If, among the stored impulses, a step is recognized the indicated coordinate is captured by the GPS connected to the microcontroller.
Afterwards, a data package with the GPS coordinate is formed, this and the time stamp is sent via the communication components of the device to a client system.
Once a step information is sent, all involved signals related to that step are eliminated from the impulse queue and all the previous signals.

9. A shoe or sneaker with an insole claim 1.
